# EUROPEAN PATENT SPECIFICATION

(11) **EP 1 151 753 B1**
(45) Date of publication and mention of the grant of the patent: **15.04.2009**
(21) Application number: 00902031.4
(22) Date of filing: 01.02.2000
(51) Int. Cl.: A61K 31/7048, A61P 9/10

(54) **PREVENTIVE AND THERAPEUTIC AGENTS FOR ARTERIOSCLEROSIS**
PROPHYLAKTISCHE UND THERAPEUTISCHE MITTEL FÜR DIE ARTERIOSKLEROSE
AGENTS PREVENTIFS ET CURATIFS CONTRE L'ARTERIOSCLEROSE

(30) Priority: 02.02.1999 JP 2539299
(43) Date of publication of application: 07.11.2001
(73) Proprietor: CCI CORPORATION, Seki-shi, Gifu 501-3923 (JP); Yoshikawa, Toshikazu, Kyoto 611-0013 (JP)
(72) Inventor: YOSHIKAWA, Toshikazu, Uji-shi, Kyoto 611-0013 (JP); MURASE, Hironobu, Gifu-shi, Gifu 502-0071 (JP); YOSHIDA, Norimasa, Kyoto-shi, Kyoto 606-0831 (JP)
(74) Representative: Lee, Nicholas John
(86) International application number: PCT/JP2000/000531
(87) International publication number: WO 2000/045824

(56) References cited:
- EP-A1- 0 611 152
- MURASE H, MOON J-H, YAMAUCHI R, KATO K, KUNIEDA T, YOSHIKAWA T, TERAO J: "Antioxidant activity of a novel vitamin E derivative, 2-(alpha-D-glucopyranosyl)methyl-2,5,7,8-t etramethylchroman-6-ol" FREE RADICAL BIOLOGY & MEDICINE, vol. 24, no. 2, 1998, pages 217-225, XP002375486
- DATABASE WPI Section Ch, Week 199748 Derwent Publications Ltd., London, GB; Class B02, AN 1997-521936 XP002377117 & JP 09 249688 A (CCI KK) 22 September 1997 (1997-09-22)
- MURASE H ET AL: "SYNTHESIS OF A NOVEL VITAMIN E DERIVATIVE, 2-(ALPHA-D-GLUCOPYRANOSYL) METHYL-2,5,7,8-TETRAMETHYLCHROMAN-6-OL, BY ALPHA-GLUCOSIDASE-CATALYZED TRANSGLYCOSYLATION" LIPIDS, CHAMPAIGN, IL, US, vol. 32, no. 1, 1997, pages 73-78, XP002918165 ISSN: 0024-4201
- DATABASE WPI Section Ch, Week 200554 Derwent Publications Ltd., London, GB; Class B02, AN 1998-348256 XP002377118 & WO 98/25629 A (CCI CORP) 18 June 1998 (1998-06-18)
- DATABASE WPI Section Ch, Week 199821 Derwent Publications Ltd., London, GB; Class B02, AN 1998-234693 XP002377119 & JP 10 072356 A (CCI KK) 17 March 1998 (1998-03-17)
- TOSHIHIKO OSAWA.: 'Lignin rui no kinousei: tokuni goma lignin wo chuushin ni' NIPPON YUKA GAKKAISHI, vol. 48, no. 10, 1999, pages 1041 - 1048, XP002927829
- WU YIH-JER, HONG CHUANG-YE, LIN SHING-JONG, WU PAULIN, SHIAO MING-SHI.: 'Increase of vitamin E content in LDL and reduction of atherosclerosis in cholesterol-fed rabbits by a water-soluble antioxidant-rich fraction of Salvia miltiorrhiza' ARTERIOSCLER. THROMB. VASC. BIOL., vol. 18, no. 3, 1998, pages 481 - 486, XP001051813
- EDSON LUIZ DA SILVA, TOJIRO TSUSHIDA AND JUNJI TERAO.: 'Inhibition of mammalian 15-lipoxygenase-dependent lipid peroxidation in low-density lipoprotein by quercetin and quercetin monoglucosides' ARCHIVES OF BIOCHEMISTRY AND BIOPHYSICS, vol. 349, no. 2, 02 January 1998, pages 313 - 320, XP002927817
- CYNSHI O ET AL.: "Antiatherogenic effects of the antioxidant BO-653 in three different animal models" PROCEEDINGS OF THE NATIONAL ACADEMY OF SCIENCES U.S.A., vol. 95, August 1998 (1998-08), pages 10123-10128,

## Description

### Technical Field

This invention relates to a novel agent for use in preventing and curing arteriosclerosis. More particularly, the invention relates to an agent for use in preventing and curing arteriosclerosis by using a water-soluble chromanol glucoside as an active component thereof.

### Background Art

The arteriosclerosis is the general germ for the localized arterial lesion which manifests itself as reconstruction, hardening, and hypofunction on the arterial wall. Among other such phenomena, the atherosclerosis which is observed to deposit on the blood vessel wall a lipid formed mainly of cholesterol is particularly important clinically. The atherosclerosis occurring in the coronary artery, the cerebral artery, the renal artery, and the artery of the limbs induces myocardial infarction, cerebral restraint, renal restraint, and necrosis of the limbs as accompanied by constriction of the canal cavity and formation of thrombosis.

Many factors are believed to be associated with the crisis and dilation of the atherosclerosis. Recently, it has been ascertained that the low specific gravity lipoprotein (LDL) which has undergone the oxidation degeneration caused by the active enzyme or the free radical is fulfilling an important role. Specifically, it is believed that during the initial stage of the atherosclerosis, numerous monocytes in the blood adhere to the hemangioendothelial cells, infiltrate the endothelia, and then succumb to differentiation into macrophage and that the scavenger acceptor of the macrophage engulfs the oxidized LDL, converts it into foam cells, and accumulates the foam cells, and gives rise to an initial atherosclerotic nest ("Medical Art of Free Radicals," written by Toshiichi Yoshikawa, published by Shindan-to-Chiryosha K.K., April 25, 1997, pp. 62-66). It is also believed that the oxidized LDL impairs the hemangioendothelial cells, disrupts the mechanism for protecting the blood vessel, and induces a process culminating in the atherosclerosis (Ross, R: The Pathogenesis of atherosclerosis: a perspective for the 1990s; Science, 362, 801-809 (1990)). In fact, the presence of the oxidized LDL has been demonstrated in vivo and the study using a monoclonal antibody against peroxidized lipoprotein has demonstrated the local presence of the oxidized LDL in the nest of the arteriosclerosis (Steinberg, D., et al.: Beyond cholesterol: Modification of law density lipoprotein that increases aetherogenicity: N. Engl. J., Med., 320: 915-925 (1989)).

From this point of view, use of an antioxidant against the arteriosclerosis has been tried. The in vitro test performed on numerous antioxidants has demonstrated that these agents are effective in inhibiting the oxidation of LDL (Sampath Parthesarathy, et al.: Probucol Inhiits Oxidative Modnfication of Law Density Lipoprotein: J. Clin. Invest., 77: 641-644 (1986) and others). Though the animal test has produced a report to the effect that the excess administration of a fat-soluble type antioxidant capable of being assimilated into the LDL to rabbits is recognized to bring a decrease in the area of the lesion of the atherosclerosis in the aorta (Cynshi. O., et al.: Antiatherogeneic effects of the antioxidant BO-6653 in three different animal models. Proc. Natl. Acad. Sci. USA., 95: 10123-10128 (1998)), this test has not given a definite answer to the question as to how such fat-soluble antioxidants are effective clinically because the LDL contains therein vitamin E, carotene, and ubiquinol which are known antioxidants from the beginning. In fact, the oxidation of the LDL is believed to be induced by the fact that the LDL migrates to below the endothelia of the blood vessels and succumbs therein to the oxidative modification caused by the hemangioendothelial cells, the smooth muscle cells of the blood vessel, and the macrophage and the active oxygen generated from such cells is believed to be associated with this oxidative modification (Steinberg, D., et al.: Beyond cholesterol: Modification of law density lipoprotein that increases aetherogenecity. N. Eng. J. Med., 320: 915-924 (1989)). Since the greater part of the oxidation of the LDL is induced by the active oxygen generated outside the LDL, it is inferred that it is important for the purpose of inhibiting the oxidation of the LDL to eliminate the active oxygen before it reacts with the LDL and stop promptly the peroxidation of lipid which has occurred on the surface of the LDL. No animal test has produced a report to the effect that the sole administration of a water-soluble antioxidant has inhibited the arteriosclerosis. It is, therefore, inferred that the conventional water-soluble antioxidant is incapable of occurring at the site fit for preventing the LDL in vivo from the active oxygen.

The chromanol glucoside which is used in this invention is a known compound (JP-A-07-118,287). This chromanol glucoside is obtained by substituting an alcohol for the phytyl group at the 2 position of a chromane ring of α-tocopherol which is a typical form of vitamin E and further binding a saccharum to the alcohol. It possesses an excellent water-solubility and an excellent antioxidative function. It has never been known to be usable for preventing and curing the arteriosclerosis.

This invention produced with a view to eliminating the problems inherent in the prior art has for an object thereof the provision of a novel agent for use in preventing and curing the arteriosclerosis, which is capable of effectively acting on the arteriosclerosis and preventing this disease from dilation at a low rate of application without entailing any secondary reaction.

Another object of this invention is to provide a novel agent for use in preventing and curing the arteriosclerosis, which can be formulated as an aqueous reagent containing the active component at a high concentration.

### Disclosure of the Invention

As a result of pursuing a diligent study regarding the pathology of the arteriosclerosis, the present inventors have found that the chromanol glucoside mentioned above dramatically prevents and improves the lesion of arteriosclerosis.

Specifically, this invention concerns an agent for use in preventing and curing the arteriosclerosis which has a chromanol glucoside represented by the following general formula (1) 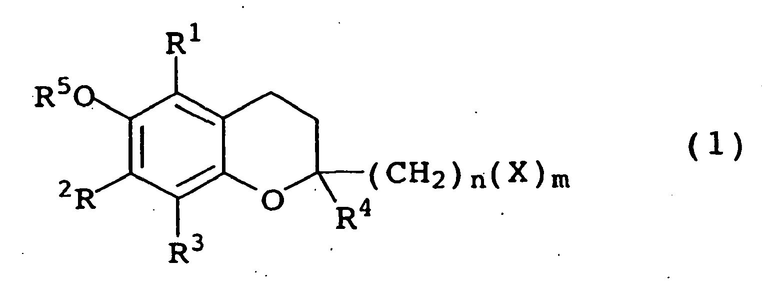 (wherein R¹, R², R³, and R⁴ are the same or different and are each a hydrogen atom or a lower alkyl group, R⁵ is a hydrogen atom, a lower alkyl group, or a lower acyl group, X is a monosaccharide residue or an oligosaccharide residue optionally having a lower alkyl group or a lower acyl group substitute for the hydrogen atom of the hydroxyl group in the saccharide residue, n is an integer in the range of 0 - 6, and m is an integer in the range of 1 - 6) as an effective component.

This invention also concerns an agent for use in preventing and curing the arteriosclerosis, wherein the chromanol glucoside mentioned above is 2-(α-D-glucopyranosyl)methyl-2,5,7,8-tetramethyl chroman-6-ol.

This invention further concerns an agent for use in preventing and curing the arteriosclerosis, wherein the arteriosclerosis mentioned above is atherosclerosis.

This invention further concerns an agent for use in preventing and curing the arteriosclerosis, wherein the agent is in the form of an aqueous preparation. Best Mode of Embodying the Invention

The agent for use in preventing and curing the arteriosclerosis contemplated by this invention is characterized by having a chromanol glucoside represented by the general formula (1) mentioned above as an active component thereof.

The lower alkyl groups in R¹, R², R³, R⁴ and R⁵ are
lower alkyl groups of 1 - 8, particularly 1 - 6, carbon atoms. As concrete examples of such lower alkyl group, a methyl group, an ethyl group, a propyl group, an isopropyl group, a butyl group, an isobutyl group, a pentyl group, an isopentyl group, a hexyl group, a heptyl group, and an octyl group may be cited. Among other groups mentioned above, the methyl group or the ethyl group prove particularly advantageous. Then, the lower acyl group in R⁵ is
a lower acyl group of 1 - 8, particularly 1 - 6, carbon atoms. As concrete examples of this lower acyl group, a formyl group, an acetyl group, a propionyl group, a butyryl group, an isobutyryl group, a valeryl group, an isovaleryl group, a pivaloyl group, a hexanoyl group, a heptanoyl group, and an octanoyl group may be cited. Among other lower acyl groups mentioned above, the acetyl group, the propionyl group, and the butylyl group prove particularly advantageous. As concrete examples of the monosaccharide residue in X, such saccharide residues as glucose, galactose, fucose, xylose, mannose, rhamnose, fructose, arabinose, lyxose, ribose, allose, altrose, idose, talose, deoxyribose, 2-deoxyribose, quinovose, and abequose may be cited. As concrete examples of the oligosaccharide residue in X, those oligosaccharide residues which are formed by combining 2 - 4 of the monosaccharides mentioned above, specifically such saccharide residues as maltose, lactose, cellobiose, raffinose, xylobiose, and sucrose may be cited. Among other oligosaccharide residues mentioned above, such monosaccharide residue as glucose, galactose, fucose, xylose, rhamnose, mannose, and fructose prove particularly advantageous. Then, the hydrogen atom of the hydroxyl group in the saccharide residue represented by X optionally may be substituted for by a lower alkyl group
of 1 - 8 carbon atoms, or a lower acyl group
of 1 - 10 carbon atoms. The letter n is an integer in the range of 0 - 6, preferably in the range of 1 - 4, and the letter m is an integer in the range of 1 - 6, preferably in the range of 1 - 3. As preferred concrete examples of the chromanol glucoside represented by the general formula (1), 2-(α-D-glucopyranosyl)methyl-2,5,7,8-tetramethylchroman-6-ol, 2-(β-D-galactopyranosyl)methyl-2,5,7,8-tetramethylchroma n-6-ol, 2-(P-fucopyranosyl)methyl-2,5,7,8.-tetramethylchroman-6-o 1, 2-(α-L-rhamnopyranosyl)-methyl-2,5,7,8-tetramethylchroma n-6-ol, 2-(β-D-xylopyranosyl)methyl-2,5,7,8-tetramethyl-chroman-6-ol, 2-(β-D-qlucopyranosyl)methyl-2,5,7,8-tetramethylchroman-6-ol, 2-(β-D-fructopyranosil)methyl-2,5,7,8-tetramethylchroman -6-oland 2-(α-D-mannopyranosyl)methyl-2,5,7,8-tetramethylchroman-6-ol may be cited.

The chromanol glucoside to be used in this invention is produced by the method disclosed in the official gazette of JP-A-07-118,287, for example, i.e. by an enzymatic reaction which comprises causing a 2-substituted alcohol represented by the following general formula (2): 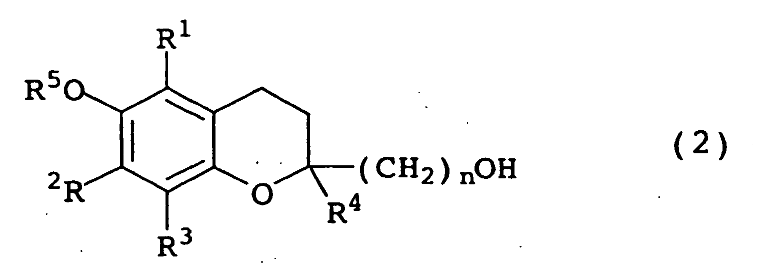 (wherein R¹, R², R³, R⁴, R⁵, and n have the same meanings as defined above), an oligosaccharide, soluble starch, starch, or cyclodextrin to react in the presence of an enzyme capable of catalyzing a corresponding transglycosiding action thereby inducing linkage of a hydroxyl group proper for the sugar specifically to the hydroxyl group at the 2 position of the 2-substituted alcohol (enzymatic method).

The 2-substituted alcohol represented by the general formula (2) and used as a raw material in the reaction described above (hereinafter referred to briefly as "2-substituted alcohol") is a known substance and is obtained by a method disclosed in the official gazette of JP-B-01-43,755 and the official gazette of JP-B-01-49,135, for example. The 2-substituted alcohol which fulfills the general formula (2) by having a methyl group for each of R¹, R², R³, and R⁴ and a hydrogen atom for R⁵ and 1 for n, for example, can be easily obtained by subjecting Trolox® to a thermal refluxing treatment in diethyl ether in the presence of hydrogenated lithium aluminum.

The enzyme which serves to catalyze the transglycosiding action in the reaction mentioned above is preferred to be used as varied with the kind of the sugar to be used in the reaction as indicated hereinbelow.
(1) In the linkage of a glucose residue with an α-bond to the 2-substituted alcohol:
   (a) The maltooligosaccharide at the position anywhere from maltose through maltotetraose is preferred to be acted on by an α-glycosidase (EC3.2.1.20). The α-glycosidase of any of nearly all origins can be used. As concrete examples of the α-glycosidase, the α-glycosidase originating in the Saccharomyces sp. produced by Toyo Spinning Co., Ltd., the α-glycosidase originating in Saccharomyces cerevisiae produced by Oriental Kobo Kogyo K.K., the α-glycosidase originating in Aspergillus niger produced by Amano Seiyaku K.K., the α-glycosidase originating in Saccharomyces sp. produced by Wako Pure Chemical Industries Ltd., the α-glycosidase originating in Bakers yeast produced by SIGMA Corp, and the α-glycosidase originating in genus Bacillus may be cited.
   (b) The soluble starch or the starch is preferred to be acted upon by 4-α-D-glucanotransferase (EC 2.4.1.25).
(2) In the linkage of a glucose residue or a maltooligo residue with an α-bond to the 2-substituted alcohol:
   The maltooligosaccharide, the soluble starch, the starch, or the cyclodextrin (α, β, γ) is preferred to be acted upon by cyclodextrin glucanotransferase (EC 2.4.1.19). As typical examples of the cyclodextrin glucanotransferase, the cyclodextrin glucanotransferase originating in Bacillus macerans produced by Amano Seiyaku K.K., the cyclodextrin glucanotransferase originating in stearothermophilus produced by Hayashibara Seibutsu Kagaku Kenkyusho K.k. and other species of cyclodextrin glucanotransferase originating in Bacillus megaterium and Bacillus circulans (ATCC 9995) may be cited.
(3) In the linkage of a glucose residue with a β-bond to the 2-substituted alcohol:
   (a) Such an oligosaccharide as cellobiose, curdlan, or laminaran which comprises a β-bond is preferred to be acted upon by β-glucosidase (EC 3.2.1.21).
   (b) The cellobiose in the presence of phosphoric acid is preferred to be acted upon by cellobiose phosphorylase (EC 2.4.1.20).
(4) In the linkage of galactose residue with an α-bond to the 2-substituted alcohol.
   (a) The melibiose or the raffinose is preferred to be acted upon by α-galactosidase (EC 3.2.1.22).
(5) In the linkage of a galactose residue with a β-bond to the 2-substituted alcohol:
   (a) The lactose or the like is preferred to be acted upon by β-galactosidase (EC 3.2.1.23).
   (b) The arabinose or the like is preferred to be acted upon by endo-1,4-β-galactanase (EC 3.2.1.89).
(6) In the linkage of a fructose residue with a β-bond to the 2-substituted alcohol.
   (a) The sucrose, the raffinose, or the melibiose is preferred to be acted upon by levansucrase (EC 2.4.1.10).
   (b) The sucrose is preferred to be acted upon by β-fructofuranosidase (EC 3.2.1.26).
   (c) The inulin or the like is preferred to be acted upon by inulin fructotransferase (EC 2.4.1.93).

The conditions to be observed in performing the reaction mentioned above are variable with the kind of chromanol glucoside and the kind of enzyme to be used. In synthesizing a chromanol glucoside which fulfills the general formula (1) by having 1 for m by the use of an α-glucosidase, for example, it is commendable to have the 2-substituted alcohol solved in advance in a sugar solution. For the sake of this solution, the addition of an organic solvent proves advantageous. As concrete examples of the organic solvent, dimethyl sulfoxide, N,N-dimethyl formamide, methanol, ethanol, acetone, and acetonitrile may be cited. When the fact of heightening the transitional activity of the α-glucosidase is additionally taken into consideration, the use of dimethyl sulfoxide or N,N-dimethyl formamide proves particularly advantageous. The concentration at which the organic solvent is added is in the range of 1 - 50 (vol/vol)%. When the efficiency of the reaction forms an important consideration, this concentration is preferred to be in the range of 5 - 35 (vol/vol)%.

The concentration of the 2-substituted alcohol is preferred to be the saturated concentration in the reaction solution or a concentration approximating closely thereto. The kinds of saccharide to be used are properly those of low molecular weights ranging approximately from maltose through maltotetraose. The preferable saccharide is maltose. The concentration of the saccharide is properly in the range of 1 - 70 (mass/vol)%, and preferably in the range of 30 - 60 (mass/vol)%. The pH is properly in the range of 4.5 - 7.5, and preferably in the range of 5.0 - 6.5. The reaction temperature is properly in the range of 10 - 70°C, and preferably in the range of 30 - 60°C. The reaction time is properly in the range of 1 - 40 hours, and preferably in the range of 2 - 24 hours. Only naturally, these reaction conditions are affected by the amount of the enzyme to be used therein. After the reaction is completed, the chromanol glucoside aimed at is obtained in high purity by treating the reaction solution by column chromatography using the product of Japan Organo K.K. as a carrier.

When a chromanol glucoside fulfilling the general formula (1) by having 1 for m is to be synthesized by using a cyclodextrin glucanotransferase, one of the reaction conditions which are observed in this synthesis is preferred to consist in solving the 2-substituted alcohol in a sugar solution. For the sake of this solution, the addition of an organic solvent proves commendable. As concrete preferred examples of the organic solvent, dimethyl sulfoxide, N,N-dimethyl formamide, methanol, ethanol, acetone, and acetonitrile may be cited. The concentration of the organic solvent to be added is properly in the range of 1 - 50 (vol/vol)%. In consideration of the efficiency of reaction, this concentration is preferably in the range of 5 - 35 (vol/vol)%. The concentration of the 2-substituted alcohol is preferred to be the saturated concentration in the reaction solution or a concentration approximating closely thereto.

As concrete preferred examples of the saccharide to be used in the reaction mentioned above, maltooligosaccharide, soluble starch, starch, and cyclodextrin (α, β, γ) which have higher degrees of polymerization than maltotriose may be cited. The concentration of this saccharide is properly in the range of 1 - 70 (mass/vol)%, and preferably in the range of 5 - 50 (mass/vol)%. The pH is properly in the range of 4.5 - 8.5, and preferably in the range of 5.0 - 7.5. The reaction temperature is properly in the range of 10 - 70°C, and preferably in the range of 30 - 60°C. The reaction time is properly in the range of 1 - 60 hours, and more preferably in the range of 2 - 50 hours. These reaction conditions, however, are affected by the amount of the enzyme to be used. The chromanol glucoside which is obtained by this reaction is a mixture fulfilling the general formula by having 1 - 8 for m. Then, by treating this mixture with glucoamylase (EC 3.2.1.3), it is made possible to obtain exclusively the chromanol glucoside which fulfills the general formula (1) by having 1 for m. The reaction temperature in this case is properly in the range of 20 - 70°C, and preferably in the range of 30 - 60°C. The reaction time is properly in the range of 0.1 - 40 hours, and preferably in the range of 1 - 24 hours. These reaction conditions, however, are affected by the amount of the enzyme to be used. Then, by subjecting the liquid remaining after the treatment with the glucoamylase mentioned above to a treatment of column chromatography using XAD, the product of Japan Organo K.K. as a carrier, it is made possible to obtain with high efficiency the chromanol glucoside which fulfills the general formula (1) by having 1 for m.

In the production of a chromanol glucoside fulfilling the general formula (1) by having 2 for m, it is made possible by causing a β-amylase (EC 3.2.1.2) to act on the chromanol glucoside produced with cyclodextrin glucanotransferase under the same conditions as described above and possessed of the form of a mixture fulfilling the general formula (1) by having 1 - 8 for m to obtain exclusively the chromanol glucoside fulfilling the general formula (1) by having 1 or 2 for m. The reaction temperature in this case is properly in the range of 20 - 70°C, and preferably in the range of 30 - 60°C. The reaction time is properly in the range of 0.1 - 40 hours, and preferably in the range of 1 - 24 hours. These reaction conditions, however, are affected by the amount of the enzyme to be used. By subjecting the liquid remaining after the treatment with the β-amylase to a treatment of column chromatography using XAD, the product of Japan Organo K.K. as a carrier, it is made possible to obtain with high purity a chromanol glycoside fulfilling the general formula (1) by having 2 for m and a chromanol glucoside fulfilling the general formula (1) by having 1 for m as well.

In the production of a chromanol glucoside fulfilling the general formula (1) by having not less than 3 for m, it is made possible by subjecting the chromanol glucoside produced with cyclodextrin glucanotransferase under the same conditions as described above and possessed of the form of a mixture fulfilling the general formula (1) by having 1 - 8 for m to a treatment as by fractionation chromatography using HPLC to obtain with high efficiency a chromanol glucoside for each of the positions of m.

The preceding mode of embodiment has described the case of linking a glucose residue or a maltooligosaccharide residue as a sugar residue to the 2-substituted alcohol. This invention can be used similarly for the mode of linking a galactose residue, a β-glucose residue, a mannose residue, or a fructose residue as a sugar residue to the 2-substituted alcohol. In this mode, the chromanol glucoside aimed at can be obtained with high purity by following the procedure of the mode described above while using a proper enzyme described in the former paragraph which has covered the catalyst capable of producing a transglycosiding action (the official gazette of JP-A-09-249,688 and the official gazette of JP-A-09-176,174).

The chromanol glucoside to be used in this invention can be also produced by subjecting the product formed by protecting the hydroxyl group at the 6 position of the 2-substituted alcohol mentioned above with a protecting group (hereinafter referred to as "saccharide acceptor") and the derivative of saccharide formed by introducing a leaving group at the anomer position and protecting the other hydroxyl group with a protecting group (hereinafter referred to as "saccharide donor") to a condensing reaction in accordance with the method described in the official gazette of JP-A-10-75,599 (organic synthetic method).

As concrete examples of the protecting group which serves to protect the hydroxyl group at the 6 position of the saccharide acceptor for use in the reaction mentioned above, an acetyl group, a benzoyl group, a pivaloyl group, a chloroacetyl group, a levulinoyl group, a benzyl group, a p-methoxybenzyl group, an allyl group, a t-butyl dimethylsilyl group, a t-butyl diphenylsilyl group, a trimethylsilyl group, and a trityl group may be cited. Among other protecting groups mentioned above, the acetyl group and the benzoyl group prove particularly advantageous.

As concrete examples of the leaving group which is introduced to the anomer position of the saccharide acceptor for use in the reaction described above, halogen atoms such as chlorine, bromine, and fluorine, sulfur compounds such as thiomethyl group, thioethyl group, and thiophenyl group, and trichloroacetoimide group may be cited. Among other leaving groups mentioned above, the bromine, the chlorine, the thiomethyl group, the thioethyl group, the thiophenyl group, and the trichloroacetaimide group prove particularly advantageous. As concrete examples of the protecting group which serves to protect the hydroxyl group at any position other than the anomer position, acyl type protecting groups such as an acetyl group, a benzoyl group, a pivaloyl group, a chloroacetyl group, and a levulinoyl group and ether type protecting groups such as a benzyl group, a p-methoxybenzyl group, an allyl group, a t-butyl dimethylsilyl group,at-butyl diphenylsilyl group, a trimethylsilyl group, and a trityl group may be cited. Among other protecting groups mentioned above, the acyl type protecting groups, especially the acetyl group, prove particularly advantageous.

The saccharide donor can be easily prepared by introducing such protecting groups to all the hydroxyl group of the saccharide and substituting leaving groups for the anomer positions by a well-known method.

To show the condensing reaction between the saccharide acceptor and the saccharide donor, the reaction is started by solving the saccharide acceptor and the saccharide donor in a nonpolar solvent. The amounts of the saccharide acceptor and the saccharide donor to be placed in the solvent are commended to have a molar ratio of the saccharide donor to the saccharide donor properly in the range of 1.0 - 1.5, and preferably in the range of 1.1 - 1.3. As concrete examples of the nonpolar solvent, methylene chloride and benzene may be cited.

Then, the condensing reaction of the saccharide donor and the saccharide acceptor is carried out in the presence of an activating agent in an anhydrous condition. As concrete examples of the activating agent, a trifluoroboric acid-ether complex, silver perchlorate, silver trifluoromethanesulfonate, mercury bromide, mercury cyanide, N-iodosuccinic acid imide-trifluoromethanesulfonic acid, dimethylmethylthiosulfonium trifurate, and p-toluenesulfonic acid may be cited. Particularly when bromine is used as a leaving group for the saccharide derivative, it is commendable to use such heavy metal salts as silver perchlorate. The reaction temperature is properly in the range of 5 - 30°C, and preferably in the range of 10 - 25°C. The reaction time is properly in the range of 12 - 48 hours, and preferably in the range of 20 - 30 hours.

Subsequently, by purifying the resultant reaction product as by silica gel column chromatography thereby denuding it of the protecting group as with sodium hydroxide and methanolic hydrochloricacid, it is made possible to obtain 2-(β-L-fucopyranosyl)methyl-2,5,7,8-tetramethyl chroman-6-ol, 2-(α-L-rhamnopyranosyl)methyl-2,5,7,8-tetramethyl chroman-6-ol, and 2-(β-D-xylopyranosyl)methyl-2,5,7,8-tetramethyl chloman-6-ol (the official gazette of JP-A-10-75,599).

The chromanol glucoside which is obtained by the enzymatic method or the organic synthesis method mentioned above is an amphoteric molecule which generally possesses exceptionally high water-solubility (about 100 g/100 ml) and abounds in oil-solubility (octanol/water distribution coefficient > 3). In other words, the chromanol glucoside which is contemplated by this invention may well be rated as a water-soluble vitamin E endowed with high affinity for fats. The chromanol-glucoside according to this invention, unlike the conventional vitamin E derivative which is insoluble or sparingly soluble in water, retains high affinity for fats even when it is used as solved in water and, therefore, penetrates cellular membranes and further infiltrates the cells, reinforces the antioxidant preventing system in the living body, prevents arteriosclerosis by effectively inhibiting and adjusting the active oxygen and the free radical in the area affected by arteriosclerosis, and brings about dramatic improvement in the morbid state of arteriosclerosis. Further, the chromanol glucoside which is obtained by the reaction described above manifests a marked improvement over tocopherol, Trolox® , or the 2-substituted alcohol in terms of thermal stability and pH stability.

The agent for use in preventing and curing arteriosclerosis according to this invention can be administered orally or non-orally to a patient in the form of a composition obtained by combining the chromanol glucoside mentioned above with a pharmaceutically allowable carrier or solving or suspending it in a pharmaceutically allowable solvent.

For the purpose of preparing this agent for oral administration, solid preparations such as tablets, dust (powder), pills, and pellets can be obtained by suitably mixing the chromanol glucoside mentioned above with proper additives such as, for example, excipients like milk sugar, mannitol, maze starch, synthetic or natural rubber, and crystalline cellulose, binding agents like starch, cellulose derivative, gum arabic, gelatin, and polyvinyl pyrrolidone, disintegrating agents like calcium carboxymethyl cellulose, sodium carboxymethyl cellulose, starch, corn starch, and sodium alginate, lustering agents like talc, magnesium stearate, and sodium stearate, and fillers or diluents like calcium carbonate, sodium carbonate, calcium phosphate, and sodium phosphate and forming the resultant mixtures in relevant shapes. These preparations may be optionally encapsulated by the use of hard or soft gelatine capsules. These solid preparations may be furnished with a coating for intestinal digestion by using coating bases such as hydroxypropyl methyl cellulose phthalate, hydroxypropyl methyl cellulose acetate succinate, cellulose acetate phthalate, and methacrylate copolymers. The chromanol glucoside mentioned above may be otherwise formed in such liquid preparations as syrups and elixirs by solving the chromanol glucoside in such an inactive diluent as purified water which is in popular use and optionally adding to the resultant solution a wetting agent, an emulsifier, a dispersion auxiliary, a surfactant, an edulcorant, a flavor, and an aromatic substance.

For the purpose of preparing the agent of this invention for use in preventing and curing arteriosclerosis for non-oral administration, the chromanol glucoside mentioned above is suitably combined with such proper buffer solutions as purified water and phosphate buffer, such physiological salt solutions as a physiological saline solution, a Ringer's solution, and a Locke's solution, ethanol, glycerin, and a surfactant in popular use and the resultant combination is sterilized to form an aqueous solution, a non-aqueous solution, a suspension, a liposome, or an emulsion, which is intravenously, hypodermically, intramuscularly, intraabdominally, intestinally, or bronchially administered preferably in the form of a sterilized aqueous solution for use with a syringe or with a spray. The liquid preparation in this case is commended to posses a physiological pH, preferably a pH in the range of 6 - 8. Optionally, the agent of this invention for preventing and curing arteriosclerosis may be administered in the form of a pellet adapted for embedment or a suppository produced by using a suppository-grade basis.

The preparation and the mode of administration which fit a given case may be selected by a physician in charge of the administration in due consideration of the information given above.

Though the concentration of the chromanol glucoside in the agent of this invention for preventing and curing arteriosclerosis is variable with such factors as the mode of administration, the kind of disease, the seriousness of the disease, and the purpose of use, it is generally in the range of 0.1 - 100 mass % and preferably in the range of 1 - 90 mass %, based on the total mass of the raw materials. It is properly in the range of 1 - 100 mass % and preferably in the range of 5 - 90 mass %, based on the total mass of the raw materials particularly when the agent is orally administered. It is properly in the range of 0.1 - 90 vol % and preferably in the range of 1 - 80 vol %, based on the total volume of the raw materials when the agent is non-orally administered. If, in this case, the concentration of the chromanol glucoside exceeds the upper limit of the range mentioned above, the excess will be at a disadvantage in failing to produce a proportionately effective improvement in the morbid state. If it falls short of the lower limit of the range, the shortage will be at a disadvantage in bringing no expected effect in the improvement of the morbid state.

The dosage of the agent of this invention for use in preventing and curing arteriosclerosis is variable with such factors as the age, body weight, and symptom of a patient, the intended mode and method of administration, the therapeutic effect aimed at, and the duration of treatment and, therefore, is left to be decided accurately by a physician in charge of agivencase. Generally, when the agent is orally administered, the dosage as reduced to the amount of the chromanol glucoside to be used is in the range of 0.1 - 10000 mg/kgofbodyweight/day. This amount is used once or as split in two or three portions daily. When the daily dosage happens to be large, the agent may be administered in the form of tablets to be taken in a plurality of pieces at a time. Then, when the agent of this invention for preventing and curing arteriosclerosis is administered non-orally, the dosage as reduced to the amount of chromanol glucoside to be used is in the range of 0.01 - 1000 mg/kg of body weight/day. This amount is used once or as split into two or three portions daily.

Now, the pharmacogenic effect of the agent of this invention for preventing and curing arteriosclerosis will be described specifically below with reference to a pharmacological test using high cholesterol-loaded rabbits and the WHHL (Watanabe heritable hyperlipidemic) rabbits forming a model of the familial high-cholesterol blood disease. The data were wholly reported by the average ± standard error (SE) and the significant differences among separate groups were determined by the Student's t-test. The risk factor, p < 0.05, was rated as forming a significance of difference.

As the chromanol glucoside, 2-(α-D-glucopyranosyl)methyl-2,5,7,8-tetramethyl chroman-6-ol (TMG) represented by the following formula (3) produced by the method described in Example 1 in the official gazette of JP-A-07-118,287 was used.

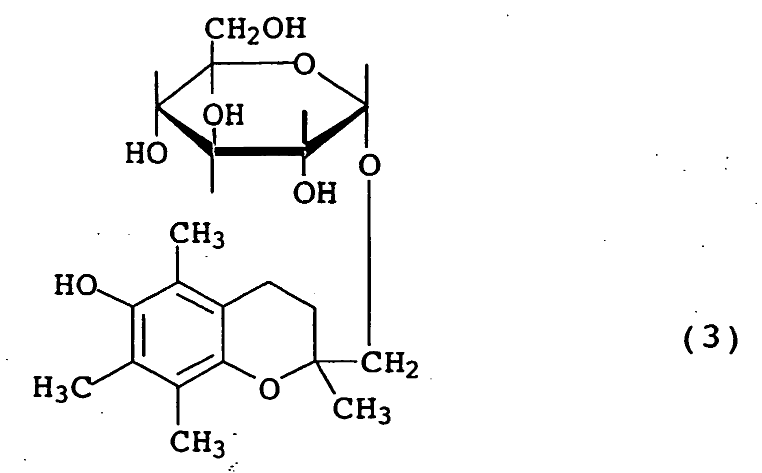

### Effect in repressing morbid alteration in the model of cholesterol-loaded rabbits

### 1. Animals used and feeding conditions

In the test, female and male New Zealand White (NZW) type rabbits (SPF: Kitayama Labels K.K.) which were seven weeks old at the time of arrival at the laboratory were subjected to quarantine and two weeks' domestication and visually inspected as to the general condition and, after the absence of abnormality was confirmed, put to use. The body weights at the time of starting administration (at the age of nine weeks) were in the range of 1.8 - 2.2 kg in the case of male rabbits and in the range of 1.6 - 2.0 kg in the case of female rabbits. Throughout the entire duration of the test, the animals were raised as accommodated one each in hanger cages of aluminum installed on a stainless steel rack inside a clean feeding chamber set at a temperature in the range of 20 - 26°, a relative humidity in the range of 40 - 70%, a number of ventilations in the range of 10 - 20 turns/hour, and an illumination time within the range of 12 hours (7.00 - 19:00). They were fed on a commercially available radioactively sterilized solid feed (a product containing 1% of cholesterol, made by Oriental Kobo K.K. and sold under the product code of "RC4") and they were left freely drinking the tap water delivered by an automatic water supplying device.

### 2. Setting dosage and method of administration

The dosage of TMG was set at 800 mg/head/day. The TMG was solved in reagent grade ethanol. The produced solution was sprayed on the 1% cholesterol-containing feed and the wet feed was left drying in the air. A mixed feed was prepared so as to contain 800 mg of TMG per 100 g of the feed. The mixed feed was given to the rabbits every morning at a rate of 100 g/day per head for 12 weeks for the sake of attaining oral consumption of the TMG. The feed for the control group was obtained by spraying ethanol alone on a feed containing 1% cholesterol and air-drying the wet feed. It was given to the rabbits of the control group at a rate of 100 g/day per heat for 12 weeks.

### 3. Formation of groups and number of animals

A control group and a TMG-administration group were formed; the control group of a total of six heads composed of three males and three females and the TMG-administration group of a total of eight heads composed of four males and four females. These animals were apportioned to the two groups randomly and nevertheless elaborately so as to avoid confusion of the children of one brood in either group to the fullest possible extent and equalize as much as permissible the average body weights and the average total cholesterol numbers in blood serum of the two groups with the body weights and the total cholesterol numbers found at the time of completion of the domestication.

### 4. Biochemical test of blood and measurement of body weight

About 6 ml of blood was taken from the heart or the auricular artery of a rabbit experiencing 12 hours' fast prior to the start of the administration, within 4 and 8 weeks of undergoing administration, and on the final day of administration (12th week). The blood was centrifuged to separate blood serum and the blood serum was analyzed for the items shown in Table 1 by the use of an automatic analyzer (made by Hitachi. Ltd. And sold under the product code of "736-20"). The body weight of each rabbit was measured at the rate of once a week throughout the entire duration of the administration. The results are shown in Table 2.

**Table 1**

| Item of test | Method of determination |
|---|---|
| Transaminase (GOT, GPT) | Formulation based on prescription in JSCC |
| Alkali phosphatase (ALP) | p-Nitrophenyl phosphoric acid substrate method |
| Lactic acid dehydrogenase (LDH) | Wroblewski-La Due method |
| Choline esterase (ChE) | Butyl thiocholine iodide substrate method |
| Albumin (ALB) | BCG method |
| Total cholesterol(T-CHO) | CHOER CHOOD EMSE method |
| Triglyceride (TG) | GK G-3-POD EMSE method |
| Phospholipid (PL) | Choline oxidase-HSDA method |

**Table 2**

| | Control group | | TMG administration group | |
|---|---|---|---|---|
| | Before administration | 12^{th} week of administration | Before administration | 12^{th} week of administration |
| Body weight (kg) | 1.92±0.08 | 3.00 ± 0.07 | 1.94 ± 0.03 | 2.89 ± 0.11 |
| GOT (mU/ml) | 20.8± 1.7 | 32.5 ± 3.9 | 20.4 ± 3.1 | 19.7±2.2* |
| GPT (mU/ml) | 38.3 ± 6.1 | 64.8 ± 8.3 | 39.5 ± 10.5 | 72.9 ± 9.1 |
| ALP (mU/ml) | 350 ± 36 | 186± 16 | 297 ± 18 | 174 ± 15 |
| LDH (mU/ml) | 153± 15 | 318 ± 99 | 132 ± 16 | 147 ± 31 |
| ChE (U/ml) | 0.15 ± 0.05 | 0.17 ± 0.06 | 0.33 ± 0.05 | 0.35 ± 0.06 |
| ALB (g/dl) | 2.3±0.1 | 2.2 ± 0.1 | 2.2 ± 0.1 | 2.3 ± 0.1 |
| T-CHO (mg/dl) | 49± 5 | 3015 ± 432 | 52 ± 5 | 2628 ± 148 |
| TG (mg/dl) | 79 ± 8 | 319 ± 87 | 64 ± 9 | 250 ± 76 |
| PL (mg/dl) | 93 ± 6 | 869 ± 103 | 94 ± 7 | 851 ± 42 |

| | | | | |
|---|---|---|---|---|
| * p < 0.05 (As compared with control group) | | | | |

### 5. Lipoprotein analysis

About 10 ml of blood was taken from the heart or the auricular artery of a rabbit experiencing 12 hours' fast prior to the start of the administration and on the 6th and 12^{th} weeks of administration. The blood was centrifuged to separate blood serum. The blood serum was ultracentrifuged by Hatch and Lees method (Hatch, F. T., and Lees, R. S. (1968) Advan, Lipid Res. 6, 1-68) using a centrifuge (the rotor; Beckman 70.1 Ti) made by Beckman and sold under the product code of "L-70") to separate low specific gravity lipoprotein (1.006 < d < 1.063), with the cholesterol amount of the LDL fraction determined by the cholesterol oxidase DASO method and the amount of HDL-cholesterol determined by subjecting the blood serum to the phospho-tungstic acid magnesium salt precipitation method. The amount of TMG was determined by the HPLC method. The results are shown in Table 3.

**Table 3**

| | | LDL-CHO (mg/dl) | HDL-CHO (mg /dl) | LDL-TMG (µg/dl) |
|---|---|---|---|---|
| Control group | Prior to administration | 17 ± 3 | 21 ± 2 | 0 |
| | 12^{th} week of administration | 630 ± 83 | 39 ± 7 | 0 |
| TMG administration group | Prior to administration | 18 ± 3 | 22 ± 2 | not detected |
| | 12^{th} week of administration | 620 ± 34 | 33 ± 5 | not detected |

### 6. Evaluation of morbid alteration of aorta

The rabbits in the 12^{th} week of administration were each subjected to median laparotomy under the anesthesia induced by the intraabdominal administration of 30 mg of Nembutal (pentobarbital sodium) per kg of body weight. The aorta ranging from the initial part of the heart through the branched part to the iliac artery in each rabbit was extracted as one specimen, incised in the direction of the major axis, pinned on a board, and fixed with neutral formalin. Then, the fixed specimen was subjected to Sudan dyeing to determine the area occupied by the deposited lipid in the whole area of aorta (surface involvement (%)) by means of an image processing software (NIH Image Freeware). The results are shown in Table 4.

**Table 4**

| | Surface involvement (%) |
|---|---|
| Control group | 79.7 ± 3.2 |
| TMG administration group | 50.2 ± 5.7** |

| | |
|---|---|
| ** p < 0.01 (as compared with the control group) | |

The control group showed a discernible rise of the GOT, an index of the development of hepatopathy, whereas the TMG administration group showed a discernible sign of significantly repressing the injury and consequently proved effective in repressing the injury. In the biochemical test of blood, the TMG administration group showed no sign of abnormality. The TMG showed no activity of lowering the blood serum lipids (T-CHO, TG, PL, LDL-CHO) which have positive connections with the arteriosclerosis and the ischemic cardiopathy. It was demonstrated to be capable of significantly repressing the occurrence of the atherosclerosis, though the existence this disease could not be recognized in the LDL. The TMG showed no activity of lowering the HDL-cholesterol which is known to have negative relations with the arteriosclerosis and the ischemic cardiopathy.

### Effect of repressing morbid alteration with WHHL rabbit model

1. Animal used and feeding conditions
In the test, female and male WHHL type rabbits (SPF: Kitayama Labels K.K.) which were eight weeks old at the time of arrival at the laboratory were subjected to quarantine and domestication and visually inspected as to the general condition and, after the absence of abnormality was confirmed, put to use. The body weights at the time of starting administration (at the age of nine weeks) were in the range of 1.5 - 1.9 kg in the case of male rabbits and in the range of 1.4 - 1.8 kg in the case of female rabbits. Throughout the entire duration of the test, the animals were raised as accommodated one each in hanger cages of aluminum installed on a stainless steel rack inside a clean feeding chamber set at a temperature in the range of 20 - 26°, a relative humidity in the range of 40 - 70%, a number of ventilations in the range of 10 - 20 turns/hour, and an illumination time within the range of 12 hours (7.00 - 19:00). They were fed on a commercially available radioactively sterilized solid feed (made by Oriental Kobo K.K. and sold under the product code of "RC4") and they were left freely drinking the tap water delivered by an automatic water supplying device.
2. Setting dosage and method of administration
The dosage of TMG was set at 800 mg/head/day. The TMG was solved in reagent grade ethanol. The produced solution was sprayed on the feed and the wet feed was left drying in the air. A mixed feed was prepared so as to contain 800 mg of TMG per 100 g of the feed. The mixed feed was given to the rabbits every morning at a rate of 100 g/day per head for 27 weeks for the sake of attaining oral consumption of the TMG. The feed for the control group was obtained by spraying ethanol alone on a feed and air-drying the wet feed. It was given to the rabbits of the control group at a rate of 100 g/day per head for 27 weeks.
3. Formation of groups and number of animals
A control group and a TMG-administration group were formed; each group consistingof threemales and three females, namely a total of six rabbits. These animals were apportioned to the two groups randomly and nevertheless elaborately so as to avoid confusion of the children of one brood in either group to the fullest possible extent and equalize as much as permissible the average body weights and the average total cholesterol numbers in blood serum of the two groups with the body weights and the total cholesterol numbers found at the time of completion of the domestication.
4. Biochemical test of blood and measurement of body weight
About 6 ml of blood was taken from the heart or the auricular artery of a rabbit experiencing 12 hours' fast prior to the start of the administration, within 4, 8, and 16 weeks of undergoing administration, and on the final day of administration (27^{th} week). The blood was centrifuged to separate blood serum and the blood serum was analyzed for the items shown in Table 5 by the use of an automatic analyzer (made by Hitachi. Ltd. and sold under the product code of "736-20"). The body weight of each rabbit was measured at the rate of once a week throughout the entire duration of the administration. The results are shown in Table 6.

**Table 5**

| Item of test | Method of determination |
|---|---|
| Transaminase (GOT, GPT) | Formulation based on prescription in JSCC |
| Alkali phosphatase (ALP) | p-Nitrophenyl phosphoric acid substrate method |
| Lactic acid dehydrogenase (LDH) | Wroblewski-La Due method |
| Choline esterase (ChE) | Butyl thiocholine iodide substrate method |
| Albumin (ALB) | BCG method |
| Total cholesterol (T-CHO) | CHOER CHOOD EMSE method |
| Triglyceride (TG) | GK G-3-POD EMSE method |
| Phospholipid (PL) | Choline oxidase-HSDA method |

**Table 6**

| | Control group | | TMG administration group | |
|---|---|---|---|---|
| | Before administration | 12^{th} week of administration | Before administration | 12^{th} week of administration |
| Body weight (kg) | 1.57 ± 0.04 | 2.92 ± 0.09 | 2.95 ± 0.07 | 2.89 ± 0.11 |
| GOT (mU/ml) | 11.5 ± 2.0 | 23.7 ± 4.5 | 20.5 ± 4.2 | 26.0 ± 11.1 |
| GPT (mU/ml) | 27.0 ± 3.0 | 49.3 ± 9.6 | 30.3 ± 2.6 | 59.7 ± 12.3 |
| ALP (mU/ml) | 314 ± 31 | 84 ± 6 | 292 ± 11 | 87± 5 |
| LDH (mU/ml) | 96 ± 10 | 116 ± 27 | 133 ± 19 | 97 ± 4 |
| ChE (U/ml) | 0.16 ± 0.05 | 0.17 ± 0.05 | 0.17 ± 0.06 | 0.19 ± 0.05 |
| ALB (g/dl) | 2.2 ± 0.1 | 2.5 ± 0.1 | 2.2 ± 0.1 | 2.4 ± 0.1 |
| T-CHO (mg/dl) | 877 ± 53 | 848± 39 | 888 ± 46 | 934 ± 49 |
| TG (mg/dl) | 254 ± 34 | 166 ± 17 | 219 ± 24 | 169 ± 23 |
| PL (mg/dl) | 464 ± 18 | 455 ± 18 | 472 ± 23 | 459 ± 52 |

| | | | | |
|---|---|---|---|---|
| * p < 0.05 (as compared with the control group) | | | | |

5. Lipoprotein analysis
About 10 ml of blood was taken from the heart or the auricular artery of a rabbit experiencing 12 hours' fast prior to the start of the administration and on the 12^{th} and 26th weeks of administration. The blood was centrifuged to separate blood serum. The blood serum was ultracentrifuged by Hatch and Lees method (Hatch, F. T., and Lees, R. S. (1968) Advan, Lipid Res. 6, 1-68) using a centrifuge (the rotor; Beckman 70.1 Ti) made by Beckman and sold under the product code of "L-70") to separate low specific gravity lipoprotein (1.006 < d < 1.063), with the cholesterol amount of the LDL fraction determined by the cholesterol oxidase DASO method and the amount of HDL-cholesterol determined by subjecting the blood serum to the phospho-tungstic acid magnesium salt precipitation method. The amount of TMG was determined by the HPLC method. The results are shown in Table 7.

**Table 7**

| | | LDL-CHO (mg/dl) | HDL-CHO (mg/dl) | LDL-TMG (µg/dl) |
|---|---|---|---|---|
| Control group | Prior to administration | 539 ± 29 | 6±1 | 0 |
| | 26^{th} week of administration | 723±33 | 5±1 | 0 |
| TMG administration group | Prior to administration | 573±35 | 6 ± 1 | not detected |
| | 26^{th} week of administration | 746±29 | 5±1 | not detected |

6. Evaluation of morbid alteration of aorta
The rabbits in the 27^{th} week of administration were each subjected to median laparotomy under the anesthesia induced by the intraabdominal administration of 30 mg of Nembutal (pentobarbital sodium) per kg of body weight. The aorta ranging from the initial part of the heart through the branched part to the iliac artery in each rabbit was extracted as one specimen, incised in the direction of the major axis, pinned on a board, and fixed with neutral formalin. Then, the fixed specimen was subjected to Sudan dyeing to determine the area occupied by the deposited lipid in the whole area of aorta (surface involvement (%)) by means of an image processing software (NIH Image Freeware). The results are shown in Table 8.

**Table 8**

| | Surface involvement (%) |
|---|---|
| Control group | 64.6±3.9 |
| TMG administration group | 52.4 ± 4.7* |

| | |
|---|---|
| * p < 0.05 (As compared with the control group) | |

In the biochemical test of blood, the TMG administration group showed no sign of abnormality. The TMG showed no activity of lowering the blood serum lipids (T-CHO, TG, PL, LDL-CHO) which have positive connections with the arteriosclerosis and the ischemic cardiopathy. It was demonstrated to be capable of significantly repressing the occurrence of the atherosclerosis, though the existence this disease could not be recognized in the LDL. The TMG showed no activity of lowering the HDL-cholesterol which is known to have negative relations with the arteriosclerosis and the ischemic cardiopathy.

### Test for acute toxicity

The agent of this invention for preventing and curing arteriosclerosis was tested for acute toxicity with a view to confirming the safety. Groups each formed of three ICR mice aged 4 to 5 weeks were used for the test. The same TMG as mentioned above was suspended as the chromanol glucoside in an aqueous 5% gum arabic solution. The suspension was orally administered to the mice at a dosage of 500 mg/kg as reduced to TMG and the mice were kept under visual inspection for one week. To the control group, the aqueous 5% gum arabic solution was orally administered at a rate of 0.3 ml. None of the mice in any of the administration groups was observed to encounter fatality.

### Production Example 1

A powder agent was obtained by mixing 100 g of TMG, 800 g of milk sugar, and 100 g of maze corn starch by the use of a blender.

### Production Example 2

A granular agent was obtained by mixing 100 g of TMG, 450 g of milk sugar, and 100 g of a low-substitution degree hydroxypropyl cellulose, then kneading the resultant mixture with 350 g of an aqueous 10% hydroxypropyl cellulose solution subsequently added thereto, extruding the produced blend and subjecting the extruded blend by the use of a pelletizer, and drying the pellets.

### Production Example 3

Tablets were obtained by mixing 100 g of TMG, 550 g of milk sugar, 215 g of maze corn starch, 130 g of crystalline cellulose, and 5 g of magnesium stearate by the use of a blender and punching the resultant mixture with a tableting machine.

### Production Example 4

Capsules were obtained by mixing 10 g of TMG, 110 g of milk sugar, 58 g of maze corn starch, and 2 g of magnesium stearate by the use of a V-shaped mixing device and filling capsules, No. 3, each with 180 mg of the resultant mixture.

### Production Example 5

An injection agent was obtained by solving 200 mg of TMG and 100 mg of glucose in 20 ml of purified water, filtering the solution, dispensing the resultant filtrate in ampoules 2 ml in volume, sealing the ampoules, then sterilizing the filled ampoules.

### Industrial Applicability

The agent of this invention for use in preventing and curing arteriosclerosis, as described above, has a water-soluble chromanol glycoside as an effective component thereof. It is, therefore, capable of reinforcing the anti-oxidant preventing system in the living body, effectively repressing and adjusting the active oxygen and the free radical in the part affected by arteriosclerosis, prominently inhibiting morbid alteration in the arteriosclerosis, and improving dramatically the state of disease.

Further, since this invention contemplates utilizing a chromanol glucoside possessed of high solubility in water as an effective component of the agent, it allows the agent to be prepared in the form of aqueous preparation containing the effective component at a high concentration and enables it to be used with unusual safety. The agent is capable of acting on the diseased part effectively at a small application rate and preventing and curing arteriosclerosis with unusually high safety because it entrains no side effect.

## Claims

1. A chromanol glucoside represented by the following general formula (1) 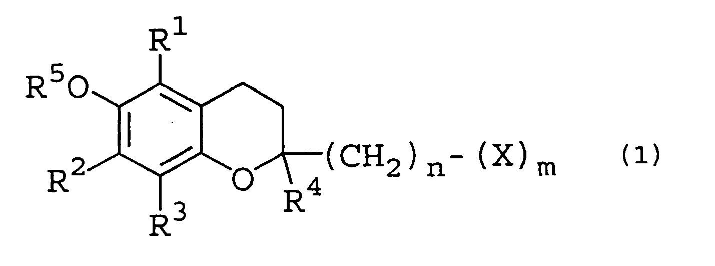 (wherein R¹, R², R³, and R⁴ are the same or different and are each a hydrogen atom or an alkyl group containing 1-8 carbon atoms, R⁵ is a hydrogen atom, an alkyl group containing 1-8 carbon atoms, or an acyl group containing 1-8 carbon atoms, X is a monosaccharide residue or an oligosaccharide residue optionally having an alkyl group containing 1-8 carbon atoms or an acyl group containing 1-10 carbon atoms substituted for the hydrogen atom of the hydroxyl group in the saccharide residue, n is an integer in the range of 0 - 6, and m is an integer in the range of 1 - 6), for use in the prevention and treatment of arteriosclerosis.

2. Use of the chromanol glucoside of claim 1 in the manufacture of a medicament for the prevention and treatment of arteriosclerosis.

3. The chromanol glucoside as claimed in claim 1, or use as claimed in claim 2, wherein said chromanol glucoside is 2-(α-D-glucopyranosyl)methyl-2,5,7,8-tetramethylchroman-6-ol.

4. The chromanol glucoside as claimed in claim 1 or claim 3, or the use as claimed in claim 2 or claim 3, wherein said arteriosclerosis is atherosclerosis.

5. The chromanol glucoside as claimed in any one of claims 1, 3, or 4, which is provided as an aqueous preparation, or the use as claimed in any one of claims 2 to 4, wherein said medicament is an aqueous preparation.

## Patentansprüche

1. Chromanolglucosid der allgemeinen Formel (1) 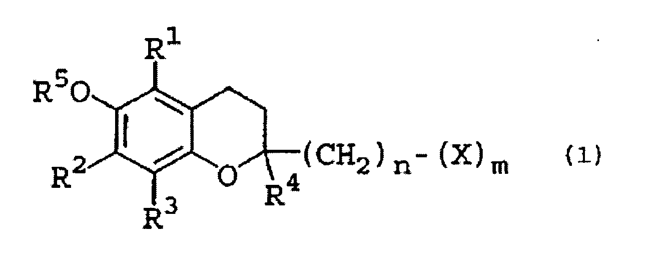 in der R¹, R², R³ und R⁴ gleich oder unterschiedlich sind und jeweils ein Wasserstoffatom oder eine Alkylgruppe mit 1-8 Kohlenstoffatomen, R⁵ ein Wasserstoffatom, eine Alkylgruppe mit 1-8 Kohlenstoffatomen oder eine Acylgruppe mit 1-8 Kohlenstoffatomen, X ein Monosaccharidrest oder ein Oligosaccharidrest, der gegebenenfalls eine Alkylgruppe mit 1-8 Kohlenstoffatomen oder eine Acylgruppe mit 1-10 Kohlenstoffatomen aufweist, die für ein Wasserstoffatom der Hydroxylgruppe in dem Saccharidrest substituiert ist, n eine ganze Zahl im Bereich von 0-6 und m eine ganze Zahl im Bereich von 1-6 bedeuten, für die Verwendung bei der Verhinderung und der Behandlung von Arteriosklerose.

2. Verwendung des Chromanolglucosids gemäß Anspruch 1 zur Herstellung eines Medikaments für die Verhinderung und die Behandlung von Arteriosklerose.

3. Chromanolglucosid gemäß Anspruch 1 oder Verwendung gemäß Anspruch 2, worin das Chromanolglucosid 2-(α-D-glucopyranosyl)methyl-2,5,7,8-tetramethylchroman-6-ol ist.

4. Chromanolglucosid nach Anspruch 1 oder 3 oder die Verwendung gemäß Anspruch 2 oder 3, wobei die Arteriosklerose Atherosklerose ist.

5. Chromanolglucosid nach jedem der Ansprüche 1, 3 oder 4, das als wässrige Zubereitung vorgelegt ist, oder die Verwendung gemäß jedem der Ansprüche 2 bis 4, wobei das Medikament eine wässrige Zubereitung ist.

## Revendications

1. Glucoside de chromanol représenté par la formule générale (1) suivante 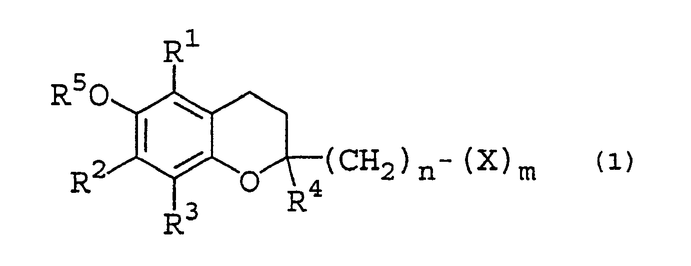 (dans laquelle R¹, R², R³, et R⁴ sont identiques ou différents et représentent chacun un atome d'hydrogène ou un groupe alkyle contenant 1 à 8 atomes de carbone, R⁵ représente un atome d'hydrogène, un groupe alkyle contenant 1 à 8 atomes de carbone, ou un groupe acyle contenant 1 à 8 atomes de carbone, X représente un résidu de monosaccharide ou un résidu d'oligosaccharide ayant éventuellement un groupe alkyle contenant 1 à 8 atomes de carbone ou un groupe acyle contenant 1 à 10 atomes de carbone substitué à l'atome d'hydrogène du groupe hydroxyle dans le résidu de saccharide, n représente un nombre entier dans la gamme de 0 à 6, et m représente un nombre entier dans la gamme de 1 à 6), pour une utilisation dans la prévention et le traitement de l'artériosclérose.

2. Utilisation du glucoside de chromanol selon la revendication 1, dans la fabrication d'un médicament destiné à la prévention et au traitement de l'artériosclérose.

3. Glucoside de chromanol selon la revendication 1, ou utilisation selon la revendication 2, où ledit glucoside de chromanol est du 2-(α-D-glucopyranosyl)méthyl-2,5,7,8-tétraméthylchroman-6-ol.

4. Glucoside de chromanol selon la revendication 1 ou la revendication 3, ou utilisation selon la revendication 2 ou la revendication 3, où ladite artériosclérose est une athérosclérose.

5. Glucoside de chromanol selon l'une quelconque des revendications 1, 3, ou 4, qui est fourni sous la forme d'une préparation aqueuse, ou utilisation selon l'une quelconque des revendications 2 à 4, où ledit médicament est une préparation aqueuse.
